# EUROPEAN PATENT SPECIFICATION

(11) **EP 2 751 275 B1**
(45) Date of publication and mention of the grant of the patent: **03.08.2016**
(21) Application number: 11764620.8
(22) Date of filing: 31.08.2011
(51) Int. Cl.: C12P 7/64

(54) **ENZYMATIC TRANSESTERIFICATION WITH LIPASES IMMOBILIZED ON HYDROPHOBIC RESINS IN WATER SOLUTIONS**
ENZYMATISCHE UMESTERUNG MIT AN HYDROPHOBEM HARZ IMMOBILISIERTEN LIPASEN IN WÄSSRIGEN LÖSUNGEN
TRANSESTÉRIFICATION ENZYMATIQUE PAR DES LIPASES IMMOBILISÉES SUR DES RÉSINES HYDROPHOBES DANS DES SOLUTIONS AQUEUSES

(43) Date of publication of application: 09.07.2014
(73) Proprietor: Trans Bio-Diesel Ltd., 20200 Shfaram (IL)
(72) Inventor: BASHEER, Sobhi, 20173 Sakhnine (IL); EGBARIEH, Ahmad, 30920 Maeleh Eron (IL); MASRI, Ramez, 20137 Nahef (IL)
(74) Representative: Srinivasan, Ravi Chandran
(86) International application number: PCT/IL2011/000699
(87) International publication number: WO 2013/030816

(56) References cited:
- EP-A1- 2 050 823
- WO-A1-2011/107977
- SALIS ET AL: "Role of the support surface on the loading and the activity of Pseudomonas fluorescens lipase used for biodiesel synthesis", JOURNAL OF MOLECULAR CATALYSIS B, vol. 57, 2009, pages 262-269, XP025991207,
- FORESTI ET AL: "Multiple effects of water on solvent-free enzymatic esterifications", ENZYME AND MICROBIAL TECHNOLOGY, vol. 41, 2007, pages 62-70, XP022069882,
- HERNANDEZ ET AL: "Simple and efficient immobilization of lipase B from Candida antarctica on porous styrene-divinylbenzene beads", ENZYME AND MICROBIAL TECHNOLOGY, vol. 49, June 2011 (2011-06), pages 72-78, XP028089866,

## Description

### Field of the Invention

Disclosed is an enzymatic process for the production of fatty acid alkyl esters for use in the biofuels, food and detergent industries. In this process a fatty acid source and an alcohol or alcohol donor are reacted in the presence of enzymes immobilized on a hydrophobic resin, in the presence of an alkaline aqueous buffer or water. The disclosed process can be operated either batchwise or continuously using a continuous stirred-tank or packed-bed column reactors.

### Background of the Invention

Immobilization of enzymes has been described by a vast number of techniques basically aiming at reducing the cost contribution of enzymes in the overall enzymatic process; facilitating recovery of enzymes from the products; and enabling continuous operation of the process.

Immobilization techniques are in general divided according to the following:
1. Physical adsorption of enzymes to solid supports, such as silica and insoluble polymers.
2. Adsorption on ion-exchange resins.
3. Covalent binding of enzymes to a solid support material, such as epoxidated inorganic or polymeric supports.
4. Entrapment of enzymes in a growing polymer.
5. Confinement of enzymes in a membrane reactor or in semi-permeable gels.
6. Cross-linking enzyme crystals (CLECS's) or aggregates (CLEAS's).

All the aforementioned enzyme immobilization procedures are comprised of the following steps:
1. Dissolving the enzyme in an appropriate buffer system with respect to pH, temperature, type of buffer salts and ionic strength.
2. Adding the solid support into the enzyme solution and mixing for some time till enzyme molecules are immobilized on the solid support.
3. Filtering off the solid support which contains the immobilized enzyme.
4. Washing the support with an appropriate buffer to remove loosely bound enzyme molecules and then drying the solid support.

Interfacial enzymes, mostly lipases, have been immobilized following the aforementioned techniques. These offered immobilized enzyme preparations possessing low synthetic activity and/or short operational half-life time. In an attempt to increase the synthetic activity and stability of immobilized lipases and other interfacial enzymes different activation methods have been applied. These methods include:
1. Binding the surface functional groups of enzymes with hydrophobic residues such as fatty acids or polyethylene glycol.
2. Coating the surface of enzymes with surfactants, such as polyol fatty acid esters.
3. Contacting enzymes with hydrophobic supports, typically polypropylene, which have been pretreated with hydrophilic solvents, such as ethanol or iso-propanol.

None of the above mentioned methods yielded satisfactory results with respect to stabilization and cost-effectiveness of immobilized interfacial enzymes, in order to carry out enzymatic reverse conversions at industrial quantities. Also, it has been reported that most enzymes, when immobilized according to the aforementioned procedures, either lose a significant portion of their synthetic activity or they do not exhibit their full activity performance due to certain constraints imposed by the immobilization procedure, or because of the presence of certain enzyme inhibitors in the reaction medium.

Another major drawback of lipases and phospholipases is their low tolerance towards hydrophilic substrates, in particular short-chain alcohols and short-chain fatty acids (below C4). It has been observed in many research studies that short-chain alcohols and short-chain fatty acids, such as methanol and acetic acid, respectively, are responsible for detaching essential water molecules from the quaternary structure of those enzymes, leading to their denaturation and consequently loss of their catalytic activity. This drawback has prohibited the application of lipases for production of commercial quantities of fatty acids methyl esters "biodiesel" using oil triglycerides and methanol as substrates.

An additional drawback of using immobilized lipases for transesterification/ esterification of a fatty acid source with a free alcohol is the accumulation of the formed glycerol and water by-products on the biocatalyst and therefore prohibiting the substrates from free access to the active site of the immobilized enzyme. Such biocatalysts generally lose their catalytic performance after a few cycles when the same batch of biocatalyst is used.

WO/2011/107977 describes an enzymatic process for the production of fatty acid alkyl esters for use in the biofuels, food and detergent industries, in which process a fatty acid source and an alcohol or alcohol donor are reacted in the presence of lipase/s immobilized on a hydrophobic resin, in the presence of an alkaline aqueous buffer or water.

US2009/0133322 describes a process for the preparation of short-chain fatty acid alkyl esters in solvent-free system, by reacting a fatty acid source with a short-chain free alcohol, in the presence of a lipase preparation which comprises at least two lipases immobilized on a suitable support, at least one of the lipases having increased affinity for partial glycerides and at least one of the lipases being sn-1,3 positional specific.

US2010/0209982 is concerned with preparations of modified lipases and phosphor-lipases, immobilized on a solid support, in which the enzyme is surrounded by hydrophobic microenvironment and is protected from deactivation and/or aggregation in the presence of hydrophilic agents, substrates and/or reaction products. The enzyme is protected by being covalently bonded with lipid groups which coat it, or by being immobilized or embedded in a hydrophobic solid support. The enzymes may be efficiently used in the preparation of biodiesel.

EP2050823 discloses a transesterification reaction where the lipase may be immobilized onto sepiolite. A base can be added to achieve a ptt between 8 and 13.

Salis et al, J. Molecular Catalysis B: Enzymatic 57 (2009) 262-269, discloses studies done to investigate the influence of the support surface on the loading and enzymatic activity of the immobilized *Pseudomonas fluorescens* lipase. There is no clear disclosure of systems comprising 5 wt% or more water.

Foresti et al, Enzyme and Microbial Technology, 41 (2007) 62-70, discloses specific polypropylene-immobilized systems which rely on a two-liquid phase reaction system.

The present inventors have developed special immobilized enzyme preparations, exhibiting good stability over many production cycles, persisting activity. Examples of such enzyme preparations are disclosed, *inter alia,* in WO/2008/084470, WO/2008/139455 and WO2009/069116.

Conditions under which the catalytic reaction is carried out, may adversely affect the stability and efficiency of immobilized enzyme preparations. It is important to have enzyme preparations which retain stability and activity under the reaction conditions.

These and other objects of the invention will become apparent as the description proceeds.

### Summary of the Invention

The present invention provides a process for the transesterification/ esterification of a fatty acid source with an alcohol, to form fatty acid alkyl esters, comprising either
(A) reacting a fatty acid source and an alcohol or an alcohol donor in the presence of an immobilized lipase preparation, wherein the immobilized lipase preparation comprises at least one lipase immobilized on a hydrophobic porous support and the reaction medium contains an aqueous alkaline buffer solution at more than 5% wt of the fatty acid source; or
(B) reacting a fatty acid source and an alcohol or an alcohol donor in the presence of an immobilized lipase preparation, wherein the immobilized lipase preparation comprises at least one lipase immobilized on a hydrophobic porous support and the reaction medium contains water at more than 5% wt of the fatty acid source.

In one embodiment, the process comprises (A) and the aqueous buffer solution has a pH from 7 to 11.

In another embodiment, the process comprises (A) and the amount of said alkaline buffer solution in the reaction medium is more than 5% wt and up to 99% wt of the fatty acid source.

In a further embodiment, the process comprises (B) and the water is in the form of a water solution of dissolved salts and the pH of the reaction system or of the water solution is from 3 to 11.

In an additional embodiment, the process comprises (B) or the process of claim 4, wherein the reaction medium contains the water or water solution of dissolved salts at more than 5% wt and up to 99% wt of the fatty acid source. Preferably, said alcohol is a short-chain C1-C6 alkyl alcohol, and said alcohol donor is a mono-alkyl ester, or a di-alkyl carbonate, serving also as a source for mild alkaline reagent in the reaction medium.

In the process of the invention the said aqueous alkaline buffer solution may be a mild aqueous alkaline buffer solution. The said aqueous alkaline buffer solution may be contained in the reaction mixture at a quantity of up to 99% wt. of the fatty acid source, for example, up to 90%, 80%, 70%, 60%, 50%, 40%, 30%, 25%, 20%, 15%, 12%, 10% and 8%. Alternatively, the said aqueous alkaline buffer solution may be contained in the reaction mixture at a quantity of more than 5% wt. of the fatty acid source, more than 6%, 8%, 10%, 12%, 15%, 20%, 25%, 30%, 40%, 50%, 60%, 70%, 80%, 90%, and up to 99%. The aqueous buffer solution may have a pH from 7 to about 11, for example any one of 7-8.5, 7-9, 7-9.5, 7-10 and 7-11. In the process of the invention, the pKa of the supplemented mild alkaline reagent comprising of the buffer solution may be higher than or equal to the pKa of free acids present in the fatty acid source.

The reaction medium of the process of the present invention may comprise water. The water is in the form of distilled water or water containing various dissolved salts, with a pH of from 3 to 11. The reaction medium may contain the water or water solution at a quantity of up to 99%wt. of the fatty acid source, for example, up to 90%, 80%, 70%, 60%, 50%, 40%, 30%, 25%, 20%, 15%, 12%, 10% and 8%. Alternatively, the water or water solution may be contained in the reaction mixture at a quantity of more than 5% wt. of the fatty acid source, more than 6%, 8%, 10%, 12%, 15%, 20%, 25%, 30%, 40%, 50%, 60%, 70%, 80%, 90%, and up to 99%.

In all embodiments and aspects of the invention, the alcohol may be a short-chain alcohol, for example C₁-C₆ alkyl alcohol, more specifically C₁-C₄ alkyl alcohol, particularly methanol or ethanol. Where said alcohol is methanol said resulting fatty acid esters are fatty acid methyl esters (FAME - Biodiesel). The alcohol may also be a medium-chain fatty alcohol (C₆-C₁₀) or long-chain fatty alcohols (C₁₂-C₂₂). The alcohol donor may be a mono-alkyl ester or a di-alkyl carbonate, such as di-methyl carbonate or diethyl carbonate.

In all embodiments and aspects of the invention, said immobilized lipase is capable of catalyzing the esterification of free fatty acids to yield fatty acid alkyl esters and water as by-product, and the transesterification of triglycerides, partial glycerides, wax esters, and phospholipids to yield fatty acid alkyl esters and glycerol, and long-chain fatty alcohols and glycerophospholipids as by-products, respectively.

In all embodiments and aspects of the invention related to the use of an alkaline buffer or alkaline solution, the amount of said alkaline buffer or solution in the reaction medium is more than 0.001% wt. of the fatty acid source.

In all embodiments and aspects of the invention, said at least one lipase may be a lipase derived from any one *Rhizomucor miehei, Pseudomonas sp., Rhizopus niveus, Mucor javanicus, Rhizopus oryzae, Aspergillus niger, Penicillium camembertii, Alcaligenes sp., Acromobacter sp., Burkholderia sp., Thermomyces lanuginosus, Chromobacterium viscosum, Candida antarctica B, Candida rugosa, Candida antarctica* A, papaya seeds and pancreatin. The lipase preparation may comprise at least two lipases which may be each separately immobilized on a hydrophobic support or co-immobilized on the same hydrophobic support. The said lipases are capable of simultaneously or consecutively catalyzing the esterification of free fatty acids to yield fatty acid alkyl esters and water as by-product, and the transesterification of triglycerides and partial glycerides to yield fatty acid alkyl esters and glycerol as by-product, and/or transesterification of phospholipids to yield fatty acid alkyl esters and lysophospholipids and glycerophospholipids as by-products.

In all embodiments and aspects of the invention, said support may be any one of hydrophobic aliphatic polymer-based support and hydrophobic aromatic polymer-based support. The said hydrophobic polymer support may be comprised of linear or branched organic chains. The said support may comprise macroreticular organic polymer or co-polymer chains. The said support may be porous or non-porous inorganic support, which may be hydrophobic or is coated with hydrophobic organic material. The said organic material may be a linear, branched, or functionalized hydrophobic organic chain.

In all embodiments and aspects of the invention where an alkaline buffer solution is used, said aqueous alkaline buffer solution may be a solution of an inorganic alkaline salt or an organic base. The said alkaline buffer solution may be a solution of any one of an alkaline metal hydroxide, carbonate, bicarbonate, phosphate, sulfate, acetate and citrate, fatty acid salts, a primary, secondary and tertiary amine, and any mixture thereof. In specific embodiments, the said alkaline buffer solution may be a solution of a weak base selected from sodium or potassium bicarbonates and carbonates. In some specific embodiments of the process of the invention, the said alkaline buffer solution may be added to said fatty acid source in a pre-mixing stage or directly to the reaction medium.

In all embodiments and aspects of the invention where an alkaline buffer solution is used, the content of said alkaline buffer solution in the transesterification/esterification reaction medium may be in an amount of more than 0.001%wt. of the oil feedstock, for example 1-30%wt., 1-20%wt., 1-10%wt., 1-5%wt. or 1-2% wt. of the oil feedstock, or amounts of more than 5%wt. of the oil feedstock, for example more than 6%, 7%, 8%, 10%, 12%, 15%, 20%, 30%, 40% and 50%wt. of the oil feedstock.

In some embodiments of the invention, the fatty acid source may be first mixed with the alkaline buffer solution or with the water or water solution, and the mixture may be then treated with said immobilized lipase preparation, followed by adding said alcohol and allowing the reaction to proceed under suitable conditions until said fatty acid source is converted to fatty acid esters.

In all embodiments and aspects of the invention said fatty acid source may be any one of plant oil, animal fat, algal oil, fish oil, waste oil and any mixtures thereof. The said fatty acid source may comprise free fatty acids, mono-, di- or tri-glycerides, their mixtures at any ratio, in the absence or presence of other minor fatty acid derivatives such as phospholipids, wax esters and sterol esters. The fatty acid source may be unrefined, refined, bleached, deodorized or any of their combinations.

In all embodiments and aspects of the invention, the reaction may be carried out at a temperature between 10°C and 100°C, specifically between 25-30°C.

In all embodiments and aspects of the invention, the said fatty acid source may be pre-mixed with said alcohol or alcohol donor and with said water or buffer solution in a pre-reaction preparation vessel to form an emulsion which may then be fed together with said immobilized lipase preparation into a transesterification/esterification reaction vessel.

In all embodiments and aspects of the invention, said immobilized lipase may be used in packed-bed column reactors operating in batch or continuous modes.

Also described herein is a system for the transesterification/esterification of a fatty acid with an alcohol, to form fatty acid alkyl esters, comprising:
a reaction vessel configured for reacting a reaction medium including a fatty acid and at least one of an alcohol and an alcohol donor in the presence of an immobilized lipase preparation, wherein the immobilized lipase preparation comprises at least one lipase immobilized on a hydrophobic porous support and the reaction medium contains at least one of an aqueous alkaline buffer solution and water.

The system may comprise one or more of the following features, in any desired combination or permutation:
A. The reaction vessel can comprise the immobilized lipase preparation, at least during operation of said system for the production of said fatty acid alkyl esters.
B. Additionally or alternatively to feature A, the reaction vessel can comprise the fatty acid and the at least one of an alcohol and an alcohol donor, at least during operation of said system for the production of said fatty acid alkyl esters.
C. Additionally or alternatively to features A or B, said reaction medium comprises a mixture, said system further comprising a pre-reaction vessel in selective fluid communication with said reaction vessel, said pre-reaction vessel being configured for premixing at least the fatty acid and the at least one of an alcohol and an alcohol donor to form said mixture, and for selectively delivering said mixture to said reaction vessel at least during operation of said system for the production of said fatty acid alkyl esters. The system can optionally further comprise a fatty acid source in selective fluid communication with said pre-reaction vessel and configured for selectively delivering the fatty acid to said pre-reaction vessel at least during said operation of said system, and an alcohol source in selective fluid communication with said pre-reaction vessel and configured for selectively delivering the at least one of an alcohol and an alcohol donor to said pre-reaction vessel at least during said operation of said system. The system can optionally further comprise a buffer source in selective fluid communication with said pre-reaction vessel and configured for selectively delivering the at least one of an aqueous alkaline buffer solution and water to said pre-reaction vessel to be included in said mixture at least during said operation of said system.
D. Additionally or alternatively to features A to C, the system can be configured for selectively delivering one or more of the fatty acid and/or the at least one of an alcohol and an alcohol donor and/or the at least one of an aqueous alkaline buffer solution and water to said pre-reaction vessel each in either a continuous manner or in discrete batches, at least during said operation of said system.
E. Additionally or alternatively to features A to D, the pre-reaction vessel can be configured for selectively delivering said mixture to said reaction vessel in a continuous manner and/or in discrete batches at least during said operation of said system.
F. Additionally or alternatively to features A to E, the system can be configured for selectively and directly delivering to said reaction vessel at least one of the fatty acid; the at least one of an alcohol and an alcohol donor; and the at least one of an aqueous alkaline buffer solution and water.
G. Additionally or alternatively to features A to F, the reaction vessel can comprise a thermal regulation system configured for maintain the reaction medium in said reaction vessel within a selected temperature range.
H. Additionally or alternatively to features A to G, the system can optionally further comprise a retaining arrangement configured for retaining the immobilized lipase preparation within said reaction vessel at least during operation of said system.
I. Additionally or alternatively to features A to H, the system further comprises a product separation vessel in selective fluid communication with said reaction vessel, said system being configured for selectively delivering a reaction mixture including reaction products from said reaction vessel to said product separation vessel, and wherein said product separation vessel is configured for selectively separating a yield of the fatty acid alkyl esters from the reaction mixture delivered thereto. For example, the product separation vessel can be one of a centrifuge and gravity separation system.
J. Additionally or alternatively to features A to I, the reaction vessel is configured for selectively delivering said reaction mixture to said product separation vessel in a continuous manner and/or in discrete batches at least during said operation of said system.
K. Additionally or alternatively to features I to J, the system is configured for selectively delivering said yield of fatty acid alkyl esters from said product separation vessel. For example, the system is configured for selectively delivering said yield of fatty acid alkyl esters from said product separation vessel in a continuous manner and/or in discrete batches.
L. Additionally or alternatively to features A to K, the system is configured for increasing said yield of the fatty acid alkyl esters from the reaction mixture delivered to said product separation vessel. In one configuration of the system having this feature, the system is configured for selectively rerouting said yield of the fatty acid alkyl esters to said reaction vessel to further increase said yield of the fatty acid alkyl esters from the reaction mixture subsequently delivered to said product separation vessel. In another configuration of the system having this feature, the system is configured for selectively rerouting said yield of the fatty acid alkyl esters to an auxiliary reactor module, wherein said auxiliary reactor module comprises an auxiliary reactor vessel and an auxiliary product separation vessel, wherein said further increased yield of the fatty acid alkyl esters is selectively subsequently delivered via said auxiliary product separation vessel.

### Brief Description of the Figures

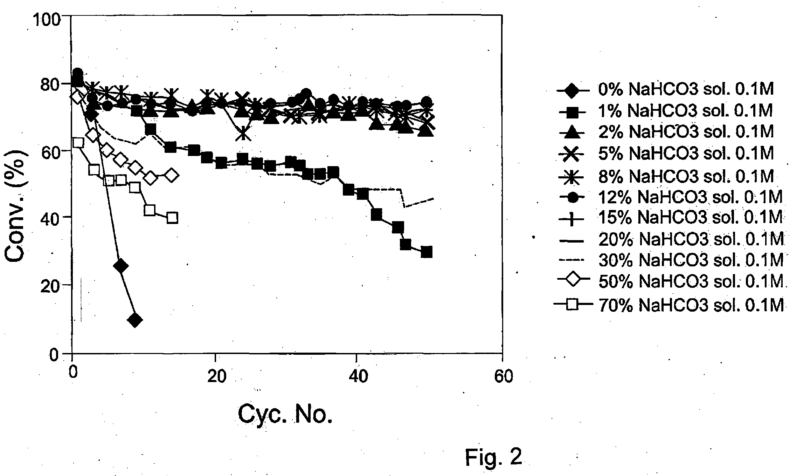

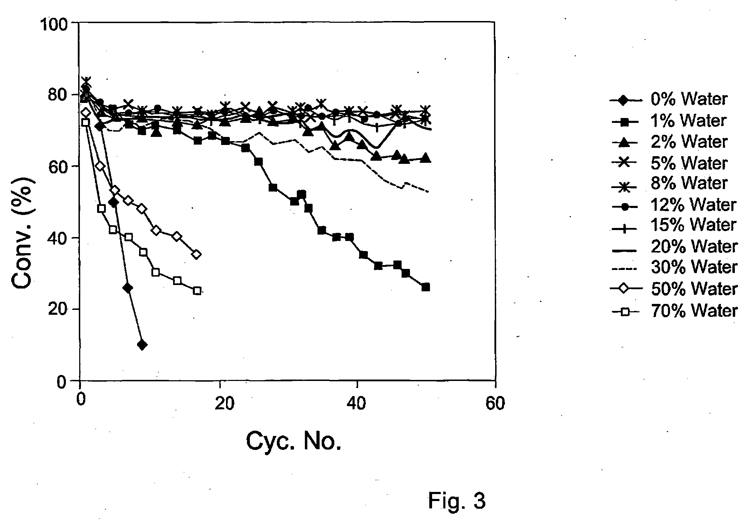

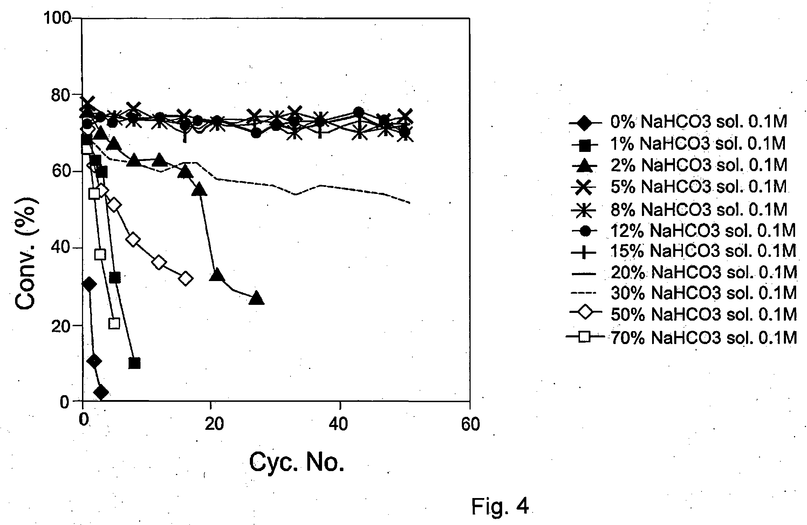

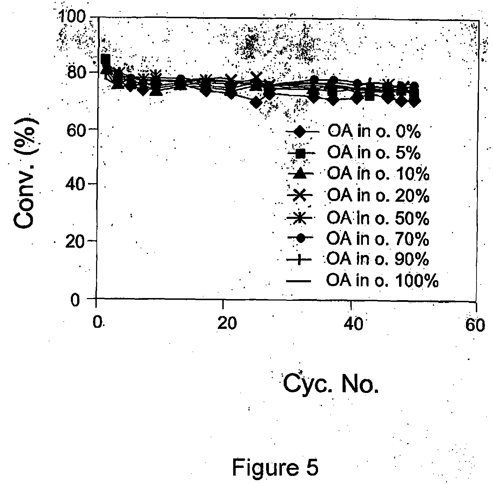

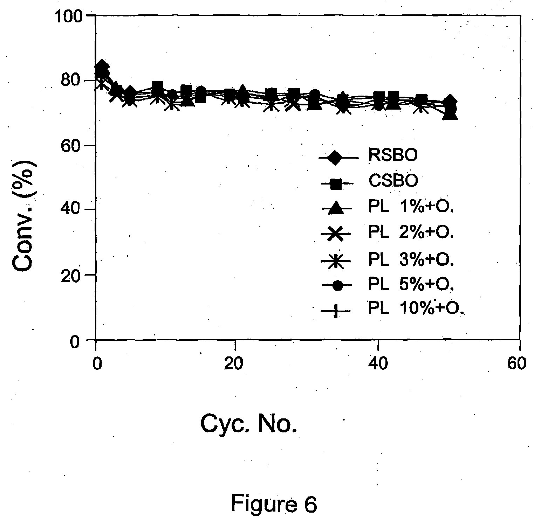

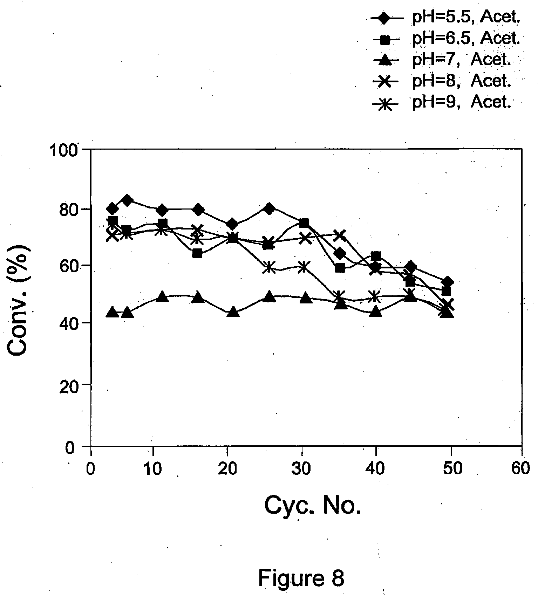

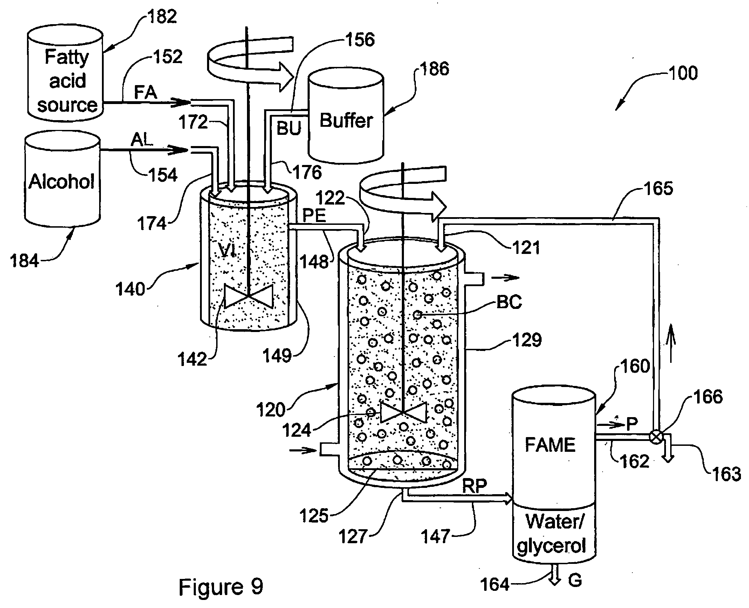

In order to understand the invention and to see how it may be carried out in practice, embodiments will now be described, by way of non-limiting example only, with reference to the accompanying drawings, in which:

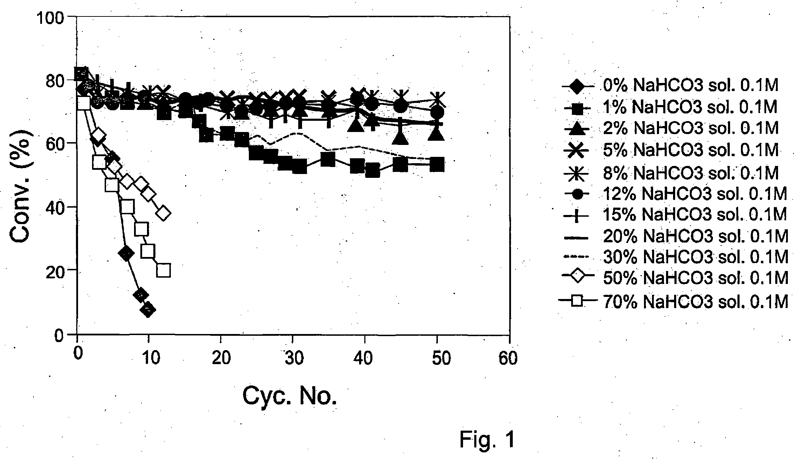

Figure 1: The conversion of soybean oil to fatty acid methyl esters and glycerol after 6 hours of reaction using the same batch of biocatalyst *(Thermomyces lanuginosus* (TL) immobilized on a DVB-PS support) in multiple batch experiments, at different concentrations of sodium bicarbonate solution of 0.1M. Methanol was added to the reaction mixture in one step on molar basis ratio of 1:3 between oil and methanol.
   Abbreviations: Conv. - conversion; Cyc. - cycle; Sol. - solution
Figure 2: The conversion of soybean oil to biodiesel and glycerol after 6 hours of reaction using the same batch of biocatalyst (*Pseudomonas sp.* (SP) immobilized on a DVB-PS support) in multiple batch experiments at different concentrations of sodium bicarbonate solution of 0.1M. Methanol was added to the reaction mixture in one step on molar basis ratio of 1:3 between oil and methanol.
   Abbreviations: Conv. - conversion; Cyc. - cycle; Sol. - solution
Figure 3: The conversion of soybean oil to biodiesel and glycerol after 6 hours of reaction using the same batch of *(Thermomyces lanuginosus* (TL) immobilized on a DVB-PS support) in multiple batch experiments at different concentrations of distilled water in the reaction mixture. Methanol was added to the reaction mixture in one step on molar basis ratio of 1:3 between oil and methanol.
   Abbreviations: Conv. - conversion; Cyc. - cycle; Wat. - water
Figure 4: The conversion of oleic acid to biodiesel and water after 6 hours of reaction using the same batch of biocatalyst (*Thermomyces lanuginosus* (TL) immobilized on a DVB-PS support) in multiple batch experiments at different concentrations of sodium bicarbonate solution of 0.1M Methanol was added to the reaction mixture in one step on molar basis ratio of 1:3 between oil and methanol.
   Abbreviations: Conv. - conversion; Cyc. - cycle; Sol. - solution
Figure 5: The conversion of different mixtures of oleic acid and soybean oil triglycerides to biodiesel, glycerol and water after 6 hours of reaction using the same batch of biocatalyst (*Thermomyces lanuginosus* (TL) immobilized on a DVB-PS support) in multiple batch experiments in the presence of 8% wt. of sodium bicarbonate solution of 0.1M. Methanol was added to the reaction mixture in one step on molar basis ratio of 1:3 between oil and methanol.
   Abbreviations: Conv. - conversion; Cyc. - cycle; Ol. Ac.. - Oleic Acid
Figure 6: The conversion of crude oils containing phospholipids to biodiesel and glycerol after 6 hours of reaction using the same batch of biocatalyst (*Thermomyces lanuginosus* (TL) immobilized on a DVB-PS support) in multiple batch experiments in the presence of 8% wt. of sodium bicarbonate solution of 0.1M Methanol was added to the reaction mixture in one step on basis of molar ratio of 1:3 between oil and methanol.
   Abbreviations: Conv. - conversion; Cyc. - cycle; RSBO - refined soybean oil; CSBO - Crude soybean oil; RSBO - refined soybean oil; PL - phospholipids; 0. - oil;

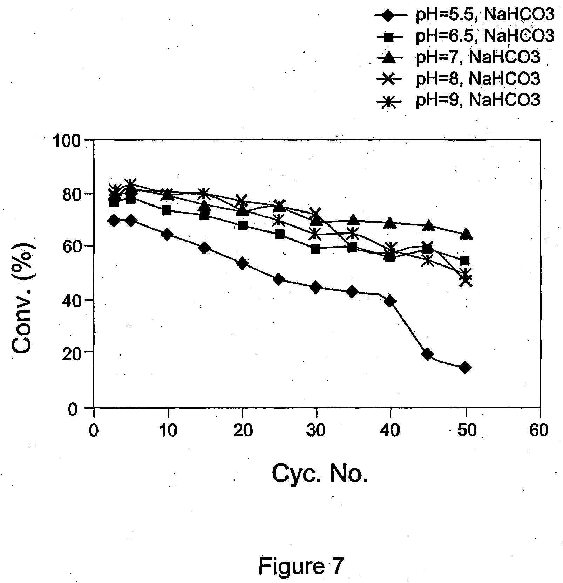

Figure 7: The conversion of soybean oil to biodiesel and glycerol after 6 hours of reaction using the same batch of (*Thermomyces lanuginosus* (TL) immobilized on a DVB-PS support) in multiple batch experiments at different pH values for sodium bicarbonate solution of 0.1M. The buffer concentration in the reaction medium was 8% wt. of the oil. Methanol was added to the reaction mixture in one step on basis of a molar ratio of 1:3 between oil and methanol.
   Abbreviations: Conv. - conversion; Cyc. - cycle
Figure 8: The conversion of soybean oil to biodiesel and glycerol after 6 hours of reaction using the same batch of biocatalyst (*Thermomyces lanuginosus* (TL) immobilized on a DVB-PS support) in multiple batch experiments at different pH values for sodium acetate solution of 0.1M. The buffer concentration in the reaction medium was 8% wt. of oil. Methanol was added in to the reaction mixture in one step on basis of a molar ratio of 1:3 between oil and methanol.
   Abbreviations: Conv. - conversion; Cyc. - cycle; Acet. - acetate
Fig. 9: illustrates schematically a system for the production of fatty + acid alkyl esters.

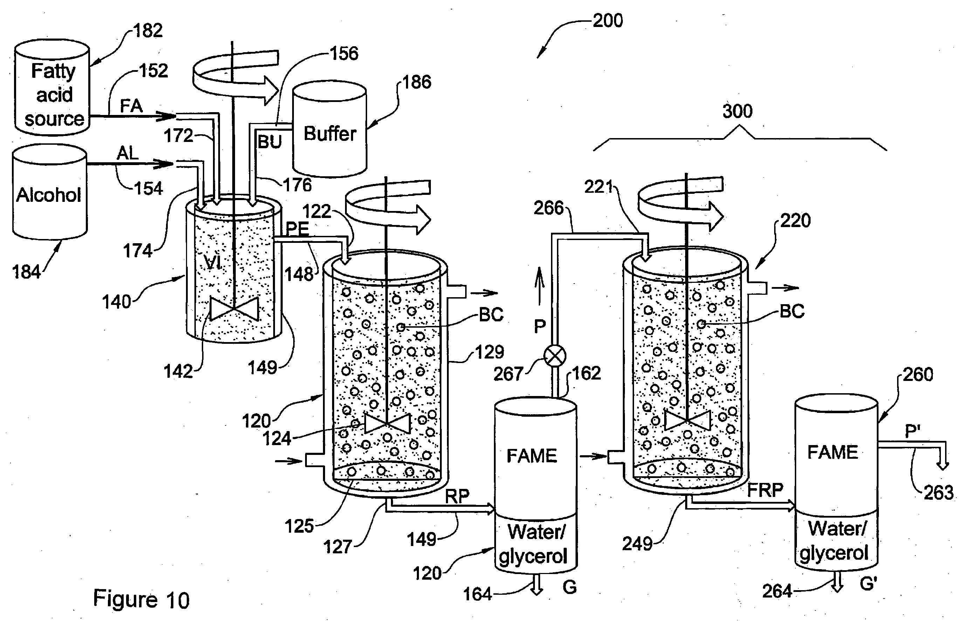

Fig. 10 illustrates schematically a system for the production of fatty acid alkyl esters.

### Detailed Description of the Invention

In search for improvement of enzymatically catalyzed industrial processes, particularly processes for transesterfication/esterification of a fatty acid source with an alcohol in the presence of immobilized lipase/s, the present inventors has developed specific conditions under which the stability of the immobilized lipase/s is preserved over scores of production cycles.

In an embodiment of the invention, the invention relates to a process for the preparation of alkyl esters of fatty acids, specifically short-chain alkyl esters of fatty acids, such as fatty acid methyl and ethyl esters (biodiesel) in a solvent-free alkaline microaqueous system. In specific embodiments, the alkaline microaqueous system is a mild alkaline microaqueous system. The process comprises providing a fatty acid source and reacting it with a free alcohol or an alcohol donor, in the presence of an immobilized lipase preparation, under said alkaline or mild alkaline conditions. Without being bound by theory, pretreatment of the fatty acid source with an alkaline buffer solution would result in neutralizing acids that might have an inhibitory effect on the enzyme. The quantity of alcohol required to complete the reaction up to 100% conversion may be added stepwise or in a one batch. Further, the alcohol may be short-chain alcohol, for example methanol or ethanol. Other alcohol donors may be used in the reaction with the fatty acid source in the presence of a hydrolase and allowing the reaction to proceed under suitable conditions, until said fatty acid source is converted to fatty acid alkyl esters, specifically, fatty acid methyl esters (FAME) or fatty acid ethyl esters, wherein said hydrolase preparation comprises one or more lipases, separately or jointly immobilized on a suitable macroreticular porous hydrophobic polymer-based support.

In an additional embodiment, the transesterification/esterification reaction between the fatty acid source and the alcohol or alcohol donor is carried out in an aqueous microenvrinment, with the addition of water to the reaction mixture. In specific embodiments, water may be added at an amount higher than 0.0001% wt. (on basis of the fatty acid source). By water as used here is meant pure or distilled water, and also "water solutions" (also referred to as aqueous solutions), which may be, but are not limited to, tap water, sea water or water from any other natural water resource or reservoir, desalinated water, chemically or enzymatically purified or treated water, and any other aqueous solutions, for example dissolved salts solutions. The pH of the reaction system or of the water solution may vary, and may be, for example, about 3-11, for example 4-10, 5-10, 5-9, 6-10, 6-9, or 7-9.

The process of the invention may be carried out while continuously removing the formed glycerol and any excess water from the reaction mixture. The conversion of the fatty acid acyl groups or free fatty acids comprised in said fatty acid source to fatty acid alkyl, specifically methyl esters may be monitored at various time points during the reaction. The reaction medium may be removed by suitable means at any desired time point during the reaction, thereby stopping the reaction, and the formed fatty acid methyl esters and optionally the formed glycerol are isolated from the reaction medium. The reaction may be specifically stopped when the conversion of the fatty acid acyl groups or free fatty acids comprised in said fatty acid source to fatty acid methyl esters has reached at least 70%, for example at least 85%, or at least 90%.

The reactions system may be similar to that described in co-pending WO2009/069116. For example, the production system may use a stirred tank reactor with a bottom sintered glass or stainless steel filter which retains the biocatalyst in the reactor, however allows the reaction medium to permeate through out of the reactor. Such reactor configuration allows by-products, specifically glycerol and water, which are self-desorbed from the immobilized enzyme, to sink to the bottom of the reactor, and permeate out through the filter. The result is continuous removal of the desorbed formed glycerol and also of excess water, out of the reaction medium, leading to shift of the reaction towards synthesis, thereby reaching conversions above 98%. The biocatalyst used in this reactor may be comprised of a single or multi-types of lipases, in consideration of their positional specificity as well as their origin, as described herein. Alternative, two consecutive stirred tank reactors with a bottom filter may be used. A settling tank or centrifuge may be used between the two reactors. The first reactor may contain an immobilized biocatalyst comprised of a single or multi-types of lipases. The role of the settling tank or centrifuge between both reactors is to remove the formed glycerol and excess water from the reaction medium, leading to an increase in the conversion of the raw materials to their corresponding fatty acid alkyl esters to above 98% in the second reactor at reasonable reaction time. Some specific reaction systems and methods are described below.

The terms "reaction mixture", "reaction system" and "reaction medium" may be used herein synonymously.

The use of lipases immobilized on hydrophobic resins in the presence of alkaline buffer solution or water, as in embodiments of the process of the invention, ensures high stability of the enzyme and also avoidance of the accumulation of hydrophilic substances, such as water and the formed glycerol by-prodcut, on the biocatalyst. In all aspects and embodiments of the process of the invention in which alkaline or mild alkaline buffer is used, it is used in more than 5% alkaline or mild alkaline buffer solution, for example, but not limited to 5-50%, 5-45%, 5-40%, 5-35%, 5-30%, 5-25%, 5-20%, 5-15%, 5-10%, 5-8%, such as but not limited to more than 5%, 6%, 7%, 8%, 10%, 12%, 15%, 20%, 25%, 30%, 35%, 40%, 45%, 50%, 60%, or 70%. Levels of the alkaline or mild alkaline buffer solution may be up to 99%wt. In all aspects and embodiments of the invention where water or water solution are used, the water or water solution is used at levels of more than 5%, for example 5-50%, 5-50%, 5-50%, 5-30%, 5-30%, 5-30%, 5-20%, 5-20%, 5-20%, such as more than 6%, 7%, 8%, 10%, 12%, 15%, 20%, 25%, 30%, 35%, 40%, 45%, 50%, or 70%. Water or water solution levels in the reaction mixture may be up to 99%wt. As mentioned, when alkaline solution is used, it may neutralize acids typically present in the fatty acid source or produced due to side reactions. Continuous active removal of these by-products may further increase the efficiency of the process. The isolated glycerol may be industrially used.

The fatty acid source used in the process of the invention may comprise at least one of soybean oil, canola oil, algae oil, rapeseed oil, olive oil, castor oil, palm oil, sunflower oil, peanut oil, cotton seed oil, Jatropha oil, crude corn oil, fish oil, animal-derived fat, waste cooking oil, brown grease, oil triglycerides derived from inedible plant sources, partial glycerides and free fatty acids derived from those oils or any mixture of at least two thereof, at any desired ratio.

An example for the use of crude oil as the fatty acid source is presented in Fig. 6, where crude soybean oil was used. This figure also shows the use of oil containing phospholipids, at various concentrations, as the fatty acid source. The use of a mixture of free fatty acids with oil is illustrated, by way of example, in Fig. 5, where a mixture of oleic acid with oil, at various concentrations, and also of oleic acid per se (100%) served as the fatty acid source.

In all processes of the invention, the fatty acid short-chain alkyl esters formed by the reaction are specifically fatty acid methyl, ethyl, *iso*-propyl or butyl esters (biodiesel). Other medium-chain fatty alcohols (C₆-C₁₀) and long-chain fatty alcohols (C₁₂-C₂₂) might also be used in the process of production of this invention. These longer alcohols may be specifically suitable in the production of waxes, for example for cosmetic products.

The lipases may be lipases derived from *Thermomyces lanuginosus, Rhizomucor miehei, Mucor miehei, Pseudomonas sp., Rhizopus sp., Mucor javanicus, Penicillium roqueforti, Aspergillus niger, Chromobacterium viscosum, Acromobacter sp., Burkholderia sp., Candida antarctica A, Candida antarctica B, Candida rugosa, Alcaligenes sp., Penicillium camembertii,* papaya seeds and pancreatin, but are not limited thereto.

The lipases may be jointly immobilized on a suitable support, specifically a hydrophobic aliphatic polymer-based support or a hydrophobic aromatic polymeric support. Each of said lipases may be immobilized on a suitable support, wherein the supports on which the said lipases are immobilized are identical or different. Lipases employed may be *regio-*specific to their substrate, or random. When more than one lipase is used, the lipases may be immobilized on the same or on different hydrophobic supports. Lipases co-immobilized on the same support can exhibit identical or different substrate selectivities or *regio*-specificities to their substrates.

Lipases may be regio-specific (or site-specific), each used alone or in combination with lipases of same or different site specificity. When referring to positions sn-1, sn-2- or sn-3, these are positions on the glycerol backbone of the various glycerides. Thus, the lipases used in the process of the invention may possess selectivity towards sn-2 position higher than that of random lipases, i.e. their favour catalyzing the reaction between the alcohol or alcohol donor with the fatty acyl group of the sn-2 position, while random lipases exhibit the same transesterification activity for fatty acyl groups at all three positions on the glycerol backbone. Some lipases uniquely exhibit positional activity on sn-2 position, especially under specific conditions determined by the substrates, products, etc. Other lipases used in the process of the invention are sn-1,3 positional specific. They may be used alone or together with a random lipase, specifically lipase that has affinity to partial glycerides, and optionally a third lipase with a high affinity to the sn-2 position.

The support is specifically a porous and macroreticular hydrophobic support, which may be organic or inorganic. Examples of supports are porous inorganic supports, such as, but not limited hydrophobized silica- or and alumina-based supports, and hydrophobic organic supports such as, but not limited to polymeric or polymer-based support. The supports may optionally contain active functional groups selected from epoxy or and aldehyde groups, or ionic groups.

The insoluble support used in the processes of the invention is specifically a porous and reticular hydrophobic aliphatic or aromatic polymer-based support, such as AmberliteR XAD 1600 and Sepabeads^{R} SP70 both comprised of porous microreticular resin prepared from divinylbenzene or from a mixture of divinylbenzene and polystyrene, Amberlite^{R} XAD 7HP comprised of microreticular aliphatic acrylic polymer, and porous aliphatic polymer such as porous polypropylene (Accurel^{R}).

The support may be a reticular hydrophobic polymer comprised of divinylbenzene, or a mixture of divinylbenzene and styrene, and reticular hydrophobic aliphatic polymer comprised of aliphatic acrylic polymers or polyalkene, such as polypropylene. Specific supports are porous matrices, of pore size in the range of 25-1000 A, and more specifically in the range of 80-200 Å. The support also may be powderous or granular porous hydrophobic silica or other inorganic oxides. The support also may be powderous or granular porous hydrophobicized silica or other inorganic oxides. In specific embodiments, the surface area of the support resins is higher than 100m²/g.

The amount of the alkaline or mild alkaline aqueous solution to be supplemented into the lipase catalyzed transesterification/ esterification reaction between the fatty acid source and the alcohol is generally adusted in accordance with the other reaction conditions, starting matrials, biocatalyst, etc. This amount can be varied, as recited and exemplified herein. This alkaline solution is prepared, for example, from an inorganic alkaline base or salt or from an organic base. Inorganic bases and salts are, for example, alkaline metal hydroxides, carbonates, bicarbonates, phosphates, sulfates, acetates and citrates. Organic bases can be, for example, primary, secondary or tertiary amines. Mixtures of these alkaline agents are also contemplated. In the process according to the invention, the pH of the microenvironment of the immobilized enzyme is maintained at alkaline or mild alkaline values. The addition of distilled water to the reaction system improves the performance of lipases immobilized on hydrophobic support (resins). As illustrated in Fig. 3, water may be added at even high quantities, while the stability of the biocatalyst (immobilized enzyme) is preserved, for example, at a water content of 30%wt., the same batch of biocatalyst exhibited 60% conversion activity after as many as 50 cycles. The addition of various alkaline buffers, with different pH values depending on the type of base used, also resulted in stabilization of lipases immobilized on hydrophobic supports (resins), as shown, for example, in Figs. 1, 2 and 4, which show that high levels of aqueous alkaline solutions did not harm the activity of the biocatalyst, with, for example, about 60% conversion rates, by the same batch of biocatalyst, at 30%wt. of 0.1M sodium bicarbonate solution in the reaction system, after more than as many as 50 cycles of the reaction. Without being bound by any theory, high concentrations of water are needed as the enzyme may preferably first hydrolyzes the ester bonds in the glyceride forms and consecutively esterify the formed free fatty acids with the supplemented alcohol. Added water also might suppress the extraction of water molecules essential to maintain the favored enzyme catalytic configuration. Carbonate and bicarbonate buffers are examples of mild bases that are efficient in increasing the stability of lipases immobilized on hydrophobic supports. Other suitable bases are described herein. Mild alkaline solution as used herein is generally a solution with a pH of from 7 to about 11, for example, 7-8.5, 7-9, 7-9.5, 7-10 or 7-11. Generally, the amount of alkaline or mild alkaline aqueous solution used is expressed by weight percents (wt.%) on basis of the amount of oil used in the reaction.

The use of lipases immobilized on porous hydrophobic polymer-based supports (resins) in the presence of an alkaline or mild alkaline solution, as well as in the presence of water or water solutions as defined herein, in the amounts recited above and also specifically exemplified, results in stabilizing the activity of the biocatalysts in the transesterification/ esterification reactions between the fatty acid source and the alcohol. This is shown in the following Examples.

The fatty acid source is at least one of triglycerides, partial glycerides, free fatty acids, phospholipids, esters and amides of fatty acids or a mixture comprised of at least two said sources.

The production of fatty acid alkyl esters is carried out by transesterification or esterification, simultaneously or sequentially. Under such reaction system the biocatalyst activity is maintained with no significant activity losses in multiple uses and also avoids the accumulation of glycerol and water by-products or other hydrophilic compounds on the biocatalyst.

This invention provides processes employing specific immobilized interfacial enzymes that retain high activity and stability over many production cycles. Specifically, lipases and phospholipases preparation are used, in transesterification/esterification reactions. These reactions may be employed in the production of food articles, cosmetics and biofuels ("biodiesel"). Of particular interest, these enzymes may be used for the synthesis of fatty acids short-chain alkyl esters for use as "biodiesel".

The present invention employed stable immobilized interfacial enzymes, of high tolerance towards short-chain alcohols, such as methanol, ethanol and glycerol, as well as short-chain fatty acids, such as acetic acid. The use of these enzyme preparations also prevents accumulation on the immobilized biocatalyst of hydrophilic substances, in particularly glycerol and water.

In an embodiment of the invention there is provided a process for simultaneous or sequential transesterfication/esterification reactions of a fatty acid source with an alcohol using one or more types of lipases, immobilized on a hydrophobic support (resin), in the presence of an alkaline or mild alkaline aqueous solution, for obtaining the desired product, namely, fatty acid alkyl esters, at near to complete conversions during reasonable reaction time, typically below 5 hours. A mild alkaline solution, for example a 0.001M, 0.1M, 0.5M or 1M solution of sodium bicarbonate, may be present in the reaction system in an amount of more than 5% wt. of the amount of oil used in the reaction, for example 6%, 8%, 10%, 12%, 15%, 20%, 25%, 30%, 40% or 50%wt.

As shown in the following Examples, the operational life time of lipases can also be extended by using hydrophobic resin support for lipase immobilization in combination with the use of an alkaline or mild alkaline buffer solution, at the various levels and ranges and sub-ranges of concentrations recited and exemplified herein, in the transesterification/esterification reaction medium. As further shown in the following Examples, the water content of the reaction mixture may be increased regardless of pH value. Thus, in another embodiment, the stability of the biocatalyst increases with increasing the water content of the reaction system by adding water, at the various levels and ranges and sub-ranges of concentrations recited and exemplified herein,. The results show that the addition of an alkaline solution (Figs. 1, 2, 4) or water (Fig. 3) results in maintaining the enzyme activity and stability over many cycles of the reaction.

The alcohol or alcohol donor employed in the processes of the invention may be a short-chain alkyl alcohol, specifically C₁-C₆ alkyl alcohol, more specifically C₁-C₄ alkyl alcohol, and particularly methanol or ethanol or the alcohol donor may be mono-alkyl ester or dialkyl carbonate, such as dimethyl carbonate. An alcohol donor such as for example dialkyl carbonate can also serve as a source for alkalinity or mild alkalinity of the reaction system.

Also described herein is a system for the production of fatty acid alkyl esters. Referring to Fig. 9, a first example of such a system, generally designated with the reference numeral **100,** comprises a reactor vessel **120,** a pre-reaction preparation vessel **140,** and a product separation vessel **160.** Pre-reaction preparation vessel 140 is configured for receiving feedstock materials and buffer (and/or water), for forming a suitable emulsion therefrom, and for feeding the prepared emulsion PE (also referred to herein as emulsified feedstock) to the reactor vessel 120. In particular, such feedback materials may include fatty acid FA (for example waste cooking oil) from a fatty acid source 182, and alcohol AL (for example methanol) from alcohol source 184, and buffer (and/or water) BU from buffer/water source 186, provided via suitable supply lines 152, 154, 156, respectively, in fluid communication with said pre-reaction preparation vessel 140 via vessel inlets 172, 174, 176, respectively and suitable valves (not shown).

The pre-reaction preparation vessel 140 defines an internal volume V1 in which the reaction mixture, including feedstock materials and buffer/water, provided therein via vessel inlets 172, 174, 176, are mixed together by means of a suitable stirring system 142, driven by a powered source (not shown), to form emulsion PE. The pre-reaction preparation vessel 140 comprises an outer jacket 149 through which a suitable work fluid may be circulated to maintain the volume V1 at a desired steady state temperature. For example, the work fluid may be oil or water, heated or cooled in a different vessel (not shown) and pumped through the jacket 149 via suitable inlet and exit ports (not shown). In alternative variations of this example, pre-reaction preparation vessel 140 may comprise a system of heating and/or cooling elements, for example electrically powered heating and/or cooling elements, instead of or in addition to the jacket 149.

Reactor vessel 120 is configured for receiving prepared emulsion PE from pre-reaction preparation vessel 140, for reacting the feedstock materials therein in the presence of a suitable biocatalyst BC to produce reaction products RP, and for feeding the reaction products RP from the reaction mixture to the product separation vessel 160. Outlet line 148 provides selective fluid communication between pre-reaction preparation vessel **140** and reactor vessel **120** via suitable valves (not shown) and allows the prepared emulsion **PE** prepared by the pre-reaction preparation vessel **140** to be fed to the reactor vessel **120** as desired.

The reaction vessel **120** defines an internal volume **V2** in which the prepared emulsion **PE** in the reaction mixture, provided therein via vessel inlet **122,** is reacted, and the reaction mixture may be stirred by means of a suitable stirring system **124,** driven by a powered source (not shown) to form the reaction products **RP.** The biocatalyst **BC** may comprise a suitable enzyme and is provided in the form of immobilized enzyme beads which remain in the reactor vessel **120** until they become ineffective or are not sufficiently effective, whereupon they may be removed and replaced with new biocatalyst **BC.** For example, the biocatalyst **BC** may comprise lipase derived from *Thermomyces lanuginosus* immobilized on a hydrophobic and porous polystyrene-divinylbenzene-based resin.

The reactor vessel **120** comprises a thermal regulation system in the form of an outer jacket **129** through which a suitable work fluid may be circulated to maintain the volume **V2** at a desired steady state temperature. For example, the work fluid may be oil or water, heated or cooled in a different vessel (not shown) and pumped through the jacket **129** via suitable inlet and exit ports **123.** In alternative variations of this example, the thermal regulation system comprises a system of heating and/or cooling elements, for example electrically powered heating and/or cooling elements, instead of or in addition to the jacket **129.**

The lower part of the reactor vessel **120** comprises an outlet **127,** and a suitable retaining arrangement in the form of filter **125** is provided upstream of the outlet **127** configured for, filtering the reaction mixture, in particular the reaction products **RP** prior to being removed from reactor vessel 120, and for preventing the biocatalyst **BC** from being removed with the reaction products **RP.**

The product separation vessel **160** is configured for separating out, from the reaction products **RP,** the desired product **P** (fatty acid alkyl ester), from by products including excess water and glycerol **G.** Outlet line **147** provides selective fluid communication between product separation vessel **160** and reactor vessel **120** via suitable valves (not shown) and allows the reaction products **RP** to be fed to the product separation vessel **160** from the reactor vessel **120** as desired. In this example, the product separation vessel **160** comprises a centrifuge or gravity separation system for carrying out the aforesaid separation, and includes a first outlet **162** for outputting the product **P,** and a second outlet **164** for collecting the excess water and glycerol **G.** Product **P** may be collected via tap **163.**

The system can thus be operated in a continuous production mode, in which prepared emulsion **PE** is fed into the reactor vessel **120,** and the desired product **P** collected in a continuous manner via tap **163.** The emulsion **PE** can be prepared and delivered in a continuous manner to the reactor vessel **120** to top up the volume of reactant therein at the same rate as the reaction products **RP** are being removed from outlet **127.** Alternatively, emulsion **PE** can be prepared and delivered in batches to the reactor vessel **120** to top up the volume of reactant in the reaction mixture at discrete intervals whenever the level of reactants in the reactor vessel **120** drops to a particular minimum level following the continuous removal of reaction products **RP** via outlet **127.** Of course, it is also possible to operate the system **100** to provide the desired product **P** in batches rather than continuously.

Alternatively, the system **100** may be operated in enhanced yield mode, wherein product **P** is, instead of being immediate collected via tap **163,** rerouted to the reactor vessel **120** via an optional rerouting system, including line **165,** vessel inlet **121** and valve **166,** wherein valve **166** may be selectively operated to divert the product **P** from tap **163.** When rerouted to reactor vessel **120,** the product **P** may be further reacted therein with alcohol **AL,** provided via a separate line (not shown) from source **184,** from a different alcohol source (not shown), or from source **184** via pre-reaction preparation vessel **140,** to produce a higher yield of product **P,** which again may be separated out from byproducts using product separation vessel **160.** When the alcohol is provided via preparation vessel **140,** the latter is first emptied of the prepared emulsion **PE,** and suitable valves prevent fatty acids **FA** and optionally buffer/water being provided by respective sourcses **182** and **186.** Suitable pumps or gravity feeds and controllable valves may be provided for selectively transporting the respective materials through the respective lines **152, 154, 156, 148, 147, 165,** and a suitable controller (not shown) monitors and controls operation of the system.

In at least some alternative variations of the first example, the pre-reaction preparation vessel **140** may be integral with the reactor vessel **120.** For example, the respective internal volumes **V1** and **V2** may be separated by a wall having an opening arrangement corresponding to the line **148.** Alternatively, the respective internal volumes **V1** and **V2** may be contiguous, but internal volume **V1** is sufficiently spaced from the biocatalyst **BC** to provide sufficient time for the emulsion **PE** to form before reaching the biocatalyst **BC.**

In alternative variations of the first example, one, two or all of the fatty acid **FA,** alcohol **AL,** and buffer/water **BU** may be provided directly to the reactor vessel **120,** bypassing the pre-reaction preparation vessel **140.** For example, one or more of the fatty acid source **182,** alcohol source **184,** and buffer/water source **186,** may be in selective fluid communication directly with reactor vessel **120** via suitable supply lines (not shown) bypassing the pre-reaction preparation vessel **140.**

It is appreciated that all components of the system **100** according to the first example, or alternative variations thereof, are of a suitable form and made from suitable materials as known in the art, such as to enable each component to carrying out the respective functions at the respective conditions, including temperature, pressure, pH and so on.

Referring to Fig. 10, a second example of the system, designated with the reference number 200, comprises all the elements and features of the first example, including alternative variations thereof, including all like-numbered components as in **Fig. 9****,** *mutatis mutandis,* with some differences. For example system **200** also comprises: a reactor vessel **120,** a pre-reaction preparation vessel **140,** a product separation vessel **160,** fatty acid source **182,** alcohol source **184,** buffer/water source **186,** supply lines **152, 154, 156,** vessel inlets **172, 174, 176,** stirring system **142,** outer jacket **149,** outlet line **148** vessel inlet **122,** stirring system **124,** biocatalyst **BC** outer jacket **129,** inlet and exit ports **123,** outlet **127,** filter **125,** outlet line **147** first outlet **162** second outlet **164;** as disclosed for the first example, *mutatis mutandis.*

However, in the second example, the line **165,** tap **163** and valve **166** of the first example are omitted, and instead an auxiliary reactor module 300 is operatively connected to the first outlet **162** of the product separation vessel **160.**

Auxiliary reactor module **300** comprises an auxiliary reactor vessel **220** and an auxiliary product separation vessel **260,** which in this example are respectively substantially similar to reactor vessel **120** and product separation vessel **160,** *mutatis mutandis.* In operation, the desired product P from product separation vessel **160** is routed to the auxiliary reactor vessel **220** via line **266,** valve **267** and vessel inlet **221.** When routed to auxiliary reactor vessel **220,** the product P may be further reacted therein with alcohol **AL,** provided via a separate line (not shown) from source **184** or from a different alcohol source (not shown), to produce further reacted products **FRP.** Line **249** enables the further reacted products **FRP** to be transported to the auxiliary product separation vessel **260,** which then operates to separate a higher yield of product P' from byproducts.

System 200 may be operated in a similar manner to system **100,** *mutatis mutandis.*

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

### Examples

### General

All experiments were carried out either in glass tubes of 30ml in volume bottomed with a centered glass filter or in mechanically stirred reactors of 500 ml in volume bottomed with a sintered glass filter of porosity of 150-250 µm. Typical reaction medium contained fatty acid source, alcohol, normally, methanol or ethanol in molar basis 1:1 in relation to the fatty acid regardless free or bound on a glycerol backbone (for free fatty acids and monoglycerides 1:1, for diglycerides 1:2, and for triglycerides 1:3 in favor of the alcohol). The fatty acid source was premixed with different amounts of alkaline buffer, in specific embodiments sodium bicarbonate. The reactions were initiated by the addition of lipase immobilized on a hydrophobic resin (10-15%wt.) and the reaction medium was either shaken mechanically or stirred at 30°C. The alcohol amount was added equally in three steps each one hour apart, unless indicated differently. Reaction conversions were followed by taking samples from the reaction medium at different time intervals and analyzing fatty acid components. The conversion to biodiesel was calculated as: 100* peak area of fatty acid alkyl ester/sum of all peaks areas.

Lipase immobilization: Lipases were immobilized following standard procedures where lipase derived from a certain microorganism is solubilized in buffer solution of 0.1M at a certain pH value, for example 7.5. An organic or inorganic polymer resin was introduced into the lipase solution. The mixture was shaken at room temperature for 8 hour. Cold acetone was optionally added into the mixture in order to increase the protein enzyme precipitation on the resin. The mixture was filtered and the enzyme beads were dried to reduce the water content to less than 5%.

Different resins were used including hydrophobic polymer resins based on polystyrene/divinylbenzen, paraffin or any of their combinations, to obtain resins of hydrophobic characteristics. Typical hydrophobic resins used included Amberlite^{R} XAD 1600 (Rohm & Haas, USA) and Sepabeads^{R} SP70 (Resindion, Italy). Typical hydrophilic resins used included Duolite^{R} D568 (Rohm & Haas) and porous silica gel. Lipases may be immobilized separately on a resin or different lipases are co-immobilized on the same resin.

### Example 1

A. The conversion of soybean oil to biodiesel and glycerol after 6 hours of reaction using the same batch of biocatalyst in multiple batch experiments.

*Reaction conditions*: Refined and bleached soybean oil (20g) containing different concentrations of sodium bicarbonate solution of 0.1M. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300 rpm and 30°C. Results are shown in Fig. 1.

B. The conversion of soybean oil to biodiesel and glycerol after 6 hours of reaction using the same batch of biocatalyst in multiple batch experiments. *Reaction conditions*: Refined and bleached soybean oil (20g) containing different concentrations of sodium bicarbonate solution of 0.1M. Methanol (2.5ml) was added in one step. Lipase derived from *Pseudomonas sp.* immobilized on a hydrophobic and porous polystyrene-divinylbenzene-based resin, was used (10%wt.). The reaction medium was shaken at 300rpm and 30°C. Results are shown in Fig. 2.

Figs. 1 and 2 show that the amount of sodium bicarbonate in the reaction medium has a major role on the operational life of lipases *Thermomyces lanuginosus* and *Pseudomonas sp.* immobilized on hydrophobic resins. It can be seen in Figs. 1 and 2 that in the absence of a mild alkaline solution both immobilized lipases drastically lost their activity after a few cycles, while the same immobilized lipases maintained their transesterification activity over multiple uses in the presence of sodium bicarbonate solution as a base in the reaction system. The results for both immobilized enzymes show that increasing the amount of sodium bicarbonate solution in the reaction medium in the range of 0 - 30% wt. results in increased enzyme transesterification activity in multiple uses of the same batch of immobilized enzyme. Increasing the amount of sodium bicarbonate solution to more than 30% wt. led to decreasing the enzyme activity. Without being bound by theory, this decrease may probably be attributed to washing out of the enzyme from the resin.

### Example 2

The conversion of soybean oil to biodiesel and glycerol after 6 hours of reaction using the same batch of biocatalyst in multiple batch experiments.

*Reaction conditions:* Refined and bleached soybean oil (20g) containing different concentrations of distilled water. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300 rpm and 30°C. Results are shown in Fig. 3.

Fig. 3 shows that the amount of water in the reaction medium also has a major role on the operational life of *Thermomyces lanuginosus lipase* immobilized on hydrophobic resins. It can be seen in Fig. 3 that in the absence of water the immobilized lipase drastically loses its activity after a few cycles, while the same immobilized lipase maintains its transesterification activity over multiple uses in the presence of water in the reaction system. The results for the immobilized enzyme show that increasing the amount of water in the reaction medium in the range of 0 - 30% wt. results in increased enzyme transesterification activity in multiple uses of the same batch of immobilized enzyme, while increasing the amount of water above 30% wt. led to decreasing the enzyme activity.

### Example 3

The conversion of oleic acid to biodiesel and water after 6 hours of reaction using the same batch of biocatalyst in multiple batch experiments.

*Reaction conditions*: Oleic acid (20g) containing different concentrations of sodium bicarbonate solution of 0.1M. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300rpm and 30°C. Results are shown in Fig. 4.

Fig. 4 shows that the concentration of sodium bicarbonate solution in the reaction medium has major role in determining the esterification activity of *Thermomyces lanuginosus* immobilized on a hydrophobic and porous polystyrene-divinylbenzene-based resin. It can be seen in Fig. 4 that in the absence of water in the reaction system the lipase immobilized on hydrophobic resin lost its activity sharply when used in multiple batch experiments. Increasing the concentration of sodium bicarbonate solution in the range of 0-20% wt. resulted in increasing the esterification activity of the biocatalyst in multiple uses. Increasing the aqueous phase amount above 30% wt resulted in loss of enzyme activity in multiple uses, most likely due to wash out of the enzyme from the resin.

### Example 4

The conversion of mixtures of oleic acid and soybean oil triglycerides to biodiesel, glycerol and water after 6 hours of reaction using the same batch of biocatalyst in multiple batch experiments.

*Reaction conditions:* Refined and bleached soybean oil containing different concentrations of oleic acid (20g) was supplemented with 8% wt. of sodium bicarbonate solution of 0.1M. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300 rpm and 30°C. Results are shown in Fig. 5.

Fig. 5 show that *Thermomyces lanuginosus* lipase immobilized on a hydrophobic and porous lipase resin and in the presence of buffer solution is capable to esterify and transesterify free fatty acids, and glycerides to form biodiesel and by-products glycerol and water. The results also show that the immobilized lipases maintain their catalytic activity with no significant activity losses in multiple uses of the same batch of biocatalyst for 50 cycles.

### Example 5

The conversion of crude oils containing phospholipids to biodiesel and glycerol after 6 hours of reaction using the same batch of biocatalyst in multiple batch experiments.

*Reaction conditions:* Crude soybean oil containing different concentrations of phospholipids (20g) was supplemented with 8% wt. of sodium bicarbonate solution of 0.1M. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300 rpm and 30°C. Results are shown in Fig. 6.

Fig. 6 shows the transesterification activity of *Thermomyces lanuginosus* lipase immobilized on a hydrophobic and porous divinylbenzene-polystyrene resin. Analysis results show in contrast to previous literature reports that lipases immobilized on hydrophobic resins in the presence of sodium bicarbonate solution are capable of transesterifying of glycerides including phospholipids to yield biodiesel, and the by-products glycerol and glycerophospholipids. Also, the results show that lipases maintain their transesterification catalytic activity when the same batch of immobilized enzyme is used in multiple uses.

### Example 6

A. The conversion of soybean oil to biodiesel and glycerol using the same batch of biocatalyst *(Thermomyces lanuginosus* (TL) immobilized on a DVB-PS support) in multiple batch experiments at different pH values for sodium bicarbonate solution of 0.1M.

*Reaction conditions:* Refined and bleached soybean oil (20g) containing 8%wt of sodium bicarbonate solution of 0.1M at different pH values. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300rpm and 30°C. Results are shown in Fig. 7.

B. The conversion of soybean oil to biodiesel and glycerol using the same batch of biocatalyst (*Thermomyces lanuginosus* (TL) immobilized on a DVB-PS support) in multiple batch experiments at different pH values for sodium acetate solution of 0.1M.

Reaction conditions: Refined and bleached soybean oil (20g) containing 8%wt of sodium acetate solution of 0.1M at different pH values. Methanol (2.5ml) was added in one step. Lipase derived from *Thermomyces lanuginosus* immobilized on a hydrophobic and porous polystyrene-divinylbenzene-based resin, was used (10%wt.). The reaction medium was shaken at 300 rpm and 30°C. Results are shown in Fig. 8.

The results presented in Fig. 7 show that at pH values of above 5.5 the biocatalyst has retained more than 60% of its initial transesterification activity after 50 cycles using the same batch of enzyme. The results show clearly that there was linear decrease in enzyme activity at pH value of 5.5 and the enzyme activity reached below 20% of the initial enzyme activity.

Similar trend has been observed when buffer acetate was used at pH values of above 6.5 where the enzyme has retained more than 50% of its initial activity after 50 repeated use (Fig. 8). The results presented in Fig. 8 show also that when sodium acetate solution of pH 5.5 was used the enzyme activity was low however maintained constant after 50 cycles of repeated use.

## Claims

1. A process for the transesterification/esterification of a fatty acid source with an alcohol, to form fatty acid alkyl esters, comprising
(A) reacting a fatty acid source and an alcohol or an alcohol donor in the presence of an immobilized lipase preparation, wherein the immobilized lipase preparation comprises at least one lipase immobilized on a hydrophobic porous support and the reaction medium contains an aqueous alkaline buffer solution at more than 5% wt of the fatty acid source; or
(B) reacting a fatty acid source and an alcohol or an alcohol donor in the presence of an immobilized lipase preparation, wherein the immobilized lipase preparation comprises at least one lipase immobilized on a hydrophobic porous support and the reaction medium contains water at more than 5% wt of the fatty acid source.

2. The process of claim 1, wherein the process comprises (A) and the aqueous buffer solution has a pH from 7 to 11.

3. The process of claim 1, wherein the process comprises (A) and the amount of said alkaline buffer solution in the reaction medium is more than 5% wt and up to 99% wt of the fatty acid source.

4. The process of claim 1, wherein the process comprises (B) and the water is in the form of a water solution of dissolved salts and the pH of the reaction system or of the water solution is from 3 to 11.

5. The process of claim 1, wherein the process comprises (B) or the process of claim 4, wherein the reaction medium contains the water or water solution of dissolved salts at more than 5% wt and up to 99% wt of the fatty acid source.

6. The process of any one of the preceding claims, wherein said alcohol is a short-chain C1-C6 alkyl alcohol, and said alcohol donor is a mono-alkyl ester, or a di-alkyl carbonate, serving also as a source for mild alkaline reagent in the reaction medium.

7. The process of any one of the preceding claims, wherein said at least one lipase is a lipase derived from any one *Rhizomucor miehei, Pseudomonas sp., Rhizopus niveus, Mucor javanicus, Rhizopus oryzae, Aspergillus niger, Penicillium camembertii, Alcaligenes sp., Acromobacter sp., Burkholderia sp., Thermomyces lanuginosus, Chromobacterium viscosum, Candida antarctica B, Candida rugosa, Candida antarctica* A, papaya seeds and pancreatin.

8. The process of any one of the preceding claims, wherein said at least one immobilized lipase is capable of catalyzing the esterification of free fatty acids to yield fatty acid alkyl esters and water as by-product, and the transesterification of triglycerides and partial glycerides to yield fatty acid alkyl esters and glycerol as by-product.

9. The process of any one of the preceding claims, wherein said lipase preparation comprises at least two lipases.

10. The process of any one of the preceding claims, wherein said support is any one of hydrophobic aliphatic polymer-based support and hydrophobic aromatic polymer-based support.

11. The process of claim 1 to 6, wherein said aqueous alkaline buffer solution is a solution of a weak base selected from sodium or potassium bicarbonates and carbonates.

12. The process of any one of the preceding claims wherein said fatty acid source is any one of plant oil, animal fat, algal oil, fish oil, waste oil and any mixtures thereof.

13. The process of any one of the preceding claims wherein said fatty acid source comprises free fatty acids, mono-, di- or tri-glycerides, their mixtures at any ratio, in the absence or presence of other minor fatty acid derivatives such as phospholipids and sterol esters, wherein said fatty acid source is unrefined, refined, bleached, deodorized or any of their combinations.

14. The process of any one of the preceding claims, wherein said alcohol is methanol and said resulting fatty acid esters are fatty acid methyl esters (FAME - Biodiesel) or said alcohol is a medium-chain fatty alcohol (C₆-C₁₀) or long-chain fatty alcohol (C₁₂-C₂₂).

## Patentansprüche

1. Verfahren zur Umesterung/Veresterung einer Fettsäurequelle mit einem Alkohol, um Fettsäurealkylester zu bilden, wobei das Verfahren Folgendes umfasst:
(A) Reagieren einer Fettsäurequelle und eines Alkohols oder eines Alkoholdonors in Gegenwart eines immobilisierten Lipasepräparats, wobei das immobilisierte Lipasepräparat mindestens eine Lipase umfasst, die auf einem hydrophoben porösen Träger immobilisiert ist, und das Reaktionsmedium eine wässrige alkalische Pufferlösung mit mehr als 5 Gew.-% der Fettsäurequelle umfasst; oder
(B) Reagieren einer Fettsäurequelle und eines Alkohols oder eines Alkoholdonors in Gegenwart eines immobilisierten Lipasepräparats, wobei das immobilisierte Lipasepräparat mindestens eine Lipase umfasst, die auf einem hydrophoben porösen Träger immobilisiert ist, und das Reaktionsmedium Wasser mit mehr als 5 Gew.-% der Fettsäurequelle umfasst.

2. Verfahren nach Anspruch 1, wobei das Verfahren (A) umfasst und die wässrige Pufferlösung einen pH-Wert von 7 bis 11 aufweist.

3. Verfahren nach Anspruch 1, wobei das Verfahren (A) umfasst und die Menge der alkalischen Pufferlösung in dem Reaktionsmedium mehr als 5 Gew.-% und bis zu 99 Gew.-% der Fettsäurequelle beträgt.

4. Verfahren nach Anspruch 1, wobei das Verfahren (B) umfasst und das Wasser in Form einer Wasserlösung von gelösten Salzen vorliegt und der pH-Wert des Reaktionssystems oder der Wasserlösung 3 bis 11 beträgt.

5. Verfahren nach Anspruch 1, wobei das Verfahren (B) umfasst oder Verfahren nach Anspruch 4, wobei das Reaktionsmedium das Wasser oder die Wasserlösung von gelösten Salzen mit mehr als 5 Gew.-% und bis zu 99 Gew.-% der Fettsäurequelle umfasst.

6. Verfahren nach einem der vorhergehenden Ansprüche, wobei der Alkohol ein kurzkettiger C₁-C₆-Alkylalkohol ist und der Alkoholdonor ein Monoalkylester oder ein Dialkylcarbonat ist, der darüber hinaus als Quelle für ein mildes alkalisches Reagenz in dem Reaktionsmedium dient.

7. Verfahren nach einem der vorhergehenden Ansprüche, wobei die mindestens eine Lipase eine Lipase ist, die von einem von Folgendem abgeleitet wird: *Rhizomucor miehei, Pseudomonas sp., Rhizopus niveus, Mucor javanicus, Rhizopus oryzae, Aspergillus niger, Penicillium camembertti, Alcaligenes sp., Acromobacter sp., Burkholderia sp., Thermomyces Ianuginosus, Chromobacterium viscosum, Candida antarctica B, Candida rugosa, Candida antarctica A,* Papayasamen und Pankreatin.

8. Verfahren nach einem der vorhergehenden Ansprüche, wobei die mindestens eine immobilisierte Lipase Folgendes katalysieren kann: die Veresterung von freien Fettsäuren, um Fettsäurealkylester und Wasser als Nebenprodukt zu ergeben, und die Umesterung von Triglyceriden und Partialglyceriden, um Fettsäurealkylester und Glycerin als Nebenprodukt zu ergeben.

9. Verfahren nach einem der vorhergehenden Ansprüche, wobei das Lipasepräparat mindestens zwei Lipasen umfasst.

10. Verfahren nach einem der vorhergehenden Ansprüche, wobei der Träger ein hydrophober aliphatischer Träger auf Polymerbasis oder ein hydrophober aromatischer Träger auf Polymerbasis ist.

11. Verfahren nach Anspruch 1 bis 6, wobei die wässrige alkalische Pufferlösung eine Lösung einer schwachen Base ist, die aus Natrium- oder Kalium-Bicarbonaten und - Carbonaten ausgewählt ist.

12. Verfahren nach einem der vorhergehenden Ansprüche, wobei die Fettsäurequelle aus einem Pflanzenöl, Tierfett, Algenöl, Fischöl, Altöl oder Mischungen davon besteht.

13. Verfahren nach einem der vorhergehenden Ansprüche, wobei die Fettsäurequelle freie Fettsäuren, Mono-, Di- oder Triglyceride, deren Mischungen in einem beliebigen Verhältnis, in Abwesenheit oder in Anwesenheit anderer geringer Mengen an Fettsäurederivaten umfasst, wie z. B. Phospholipide und Sterolester, wobei die Fettsäurequelle nicht raffiniert, raffiniert, gebleicht, desodoriert ist oder mit Kombinationen davon behandelt wurde.

14. Verfahren nach einem der vorhergehenden Ansprüche, wobei der Alkohol Methanol ist und die resultierenden Fettsäureester Fettsäuremethylester (Fatty Acid Methyl Ester, FAME - Biodiesel) sind oder der Alkohol ein mittelkettiger Fettalkohol (C₆-C₁₀) oder ein langkettiger Fettalkohol (C₁₂-C₂₂) ist.

## Revendications

1. Procédé de transestérification/estérification d'une source d'acide gras avec un alcool, pour former des esters alkyliques d'acides gras, comprenant :
(A) la réaction d'une source d'acide gras et d'un alcool, ou d'un donneur d'alcool, en présence d'une préparation de lipase immobilisée, où la préparation de lipase immobilisée comprend au moins une lipase immobilisée sur un support poreux hydrophobe et le milieu réactionnel contient une solution tampon alcaline aqueuse à plus de 5 % en masse par rapport à la masse de source d'acide gras ; ou
(B) la réaction d'une source d'acide gras et d'un alcool ou d'un donneur d'alcool, en présence d'une préparation de lipase immobilisée, où la préparation de lipase immobilisée comprend au moins une lipase immobilisée sur un support poreux hydrophobe et le milieu réactionnel contient de l'eau à plus de 5 % en masse par rapport à la masse de source d'acide gras.

2. Procédé selon la revendication 1, dans lequel le procédé comprend (A) et la solution tampon aqueuse a un pH compris entre 7 et 11.

3. Procédé selon la revendication 1, dans lequel le procédé comprend (A) et la quantité de ladite solution tampon alcaline dans le milieu réactionnel est à plus de 5 % en masse et jusqu'à 99 % en masse par rapport à la masse de source d'acide gras.

4. Procédé selon la revendication 1, dans lequel le procédé comprend (B) et l'eau est sous la forme d'une solution aqueuse de sels dissous et le pH du système réactionnel ou de la solution aqueuse est compris entre 3 et 11.

5. Procédé selon la revendication 1, dans lequel le procédé comprend (B) ou le procédé selon la revendication 4, dans lequel le milieu réactionnel contient de l'eau ou une solution aqueuse de sels dissous à plus de 5 % en masse et jusqu'à 99 % en masse par rapport à la masse de la source d'acide gras.

6. Procédé selon l'une quelconque des revendications précédentes, dans lequel ledit alcool est un alcool alkylique à courte chaîne de Ci à C₆, et ledit donneur d'alcool est un ester monoalkylique, ou un carbonate de dialkyle, servant également de source de réactif modérément alcalin dans le milieu réactionnel.

7. Procédé selon l'une quelconque des revendications précédentes, dans lequel ladite au moins une lipase est une lipase dérivée de l'une quelconque parmi : *Rhizomucor miehei, Pseudomonas sp., Rhizopus niveus, Mucor javanicus, Rhizopus oryzae, Aspergullus niger, Penicilium camembertii, Alcaligenes sp., Acromobacter sp., Burkholderia sp., Thermomyces lanuginosus, Chromobacterium viscosum, Candida antarctica B, Candida ruigosa, Candida antarctica A,* graines de papaye et pancréatine.

8. Procédé selon l'une quelconque des revendications précédentes, dans lequel ladite au moins une lipase immobilisée est susceptible de catalyser l'estérification des acides gras libres pour produire des esters alkyliques d'acide gras et de l'eau en tant que sous-produit, et la transestérification des triglycérides et des glycérides partiels pour produire des esters alkyliques d'acide gras et du glycérol en tant que sous-produit.

9. Procédé selon l'une quelconque des revendications précédentes, dans lequel ladite préparation de lipase comprend au moins deux lipases.

10. Procédé selon l'une quelconque des revendications précédentes, dans lequel ledit support est un support quelconque parmi un support à base d'un polymère aliphatique hydrophobe et un support à base d'un polymère aromatique hydrophobe.

11. Procédé selon les revendications 1 à 6, dans lequel ladite solution tampon alcaline aqueuse est une solution d'une base faible choisie parmi les carbonates et bicarbonates de potassium et de sodium.

12. Procédé selon l'une quelconque des revendications précédentes, dans lequel ladite source d'acide gras est un corps gras quelconque parmi une huile végétale, une graisse animale, une huile d'algue, une huile de poisson, une huile usagée et un mélange quelconque de celles-ci.

13. Procédé selon l'une quelconque des revendications précédentes, dans lequel ladite source d'acide gras comprend les acides gras libres, les mono, di ou triglycérides, leurs mélanges dans des proportions quelconques, en l'absence ou en présence d'autres dérivés mineurs d'acides gras comme des phospholipides et des esters de stérol, où ladite source d'acide gras est brute, raffinée, décolorée, désodorisée ou est une quelconque combinaison de ceux-ci.

14. Procédé selon l'une quelconque des revendications précédentes, dans lequel ledit alcool est du méthanol et lesdits esters d'acide gras obtenus sont des esters méthyliques d'acide gras (EMAG - Biodiesel) ou ledit alcool est un alcool gras à chaîne intermédiaire (de C₆ à C₁₀) ou un alcool gras à longue chaîne (de C₁₂ à C₂₂).
